# EUROPEAN PATENT SPECIFICATION

(11) **EP 4 414 019 B1**
(45) Date of publication and mention of the grant of the patent: **09.12.2026**
(21) Application number: 24169251.6
(22) Date of filing: 09.04.2024
(51) Int. Cl.: A61M 39/22

(54) **IMPROVED VALVE, PARTICULARLY OF THE TYPE FOR MEDICAL USE**
VERBESSERTES VENTIL, INSBESONDERE FÜR MEDIZINISCHE ZWECKE
VALVE AMÉLIORÉE, EN PARTICULIER DU TYPE À USAGE MÉDICAL

(43) Date of publication of application: 14.08.2024
(73) Proprietor: Sganzaroli, Renato, 20094 Corsico (MI) (IT); Sganzaroli, Massimo, 20089 Rozzano (MI) (IT)
(72) Inventor: Sganzaroli, Renato, 20094 Corsico (MI) (IT); Sganzaroli, Massimo, 20089 Rozzano (MI) (IT)
(74) Representative: Modiano, Micaela Nadia

(56) References cited:
- AU-A1- 2018 204 614
- JP-A- H11 342 209
- US-A1- 2017 281 922

## Description

The present invention relates to an improved valve, particularly of the type for medical use.

Nowadays, there are many and varied types of medical valves on the market.

Although they meet current regulations, for example relating to health security, to operating tests and/or to the shape structure of some of their parts, they differ in dimensions, shapes and/or grips.

More specifically, the medical valves on the market have a plurality of inlets and outlets and a flow control element adapted to intercept and/or deviate the flow of a fluid inside one or more of such inlets and/or outlets.

In order to regulate the direction of the flow and the communication between such inlets and outlets, one particular type of conventional medical valve comprises a flow control element provided with a plurality of slots on its outer surface and proximate to/at its head, i.e. an upper portion thereof adapted to interface with a portion of the valve body, into which such flow control element is inserted, and provided with a protrusion that extends from the inner walls of the chamber.

This protrusion is contoured and adapted to be accommodated inside the cavity defined by the slots on the head of the flow control element, following a relative rotation of the latter with respect to the valve body.

Such conventional valves are not devoid of drawbacks, however.

In fact, when the relative rotation occurs between the flow control element and the valve body to pass from a first configuration of use to a second configuration of use of the valve, the protrusion on the inner walls of the chamber exerts a pressure on the head of the valve body, particularly on a portion comprised between two successive slots.

This pressure causes the head of the flow control element to deform elastically and, during the transition between two successive configurations, a proper hydraulic seal is not achieved, so leading to possible leaks of fluid.

The elastic deformation of the head owing to the contact with the protrusion generates a leak route of the fluid, which can exit from the upper part of the valve body and, if the valve body is open at the bottom, also from its lower part. Patent documents AU2018204614A1, US2017/281922A1, and JPH11342209 disclose features falling under the preamble of claim 1.

The aim of the present invention is to provide an improved valve, particularly of the type for medical use, that is capable of overcoming the above-mentioned drawbacks.

Within this aim, an object of the present invention consists in providing a valve that is capable of regulating the passage of one or more fluids and which is free from leaks in any condition for use of the valve.

Another object of the present invention consists in providing a valve that is easy to use and simple from the point of view of construction.

A further object of the invention is to provide a valve for which the molding process produces no waste material, for which recycling is not permitted and which therefore cannot be reused.

Another object of the present invention consists in providing a valve that is capable of offering the widest assurances of reliability, efficiency and operation.

This aim and these and other objects which will become more apparent hereinafter are achieved by an improved valve, particularly of the type for medical use, according to claim 1.

Further characteristics and advantages of the invention will become more apparent from the description of two preferred, but not exclusive, embodiments of an improved valve, particularly of the type for medical use, which are illustrated by way of non-limiting example with the aid of the accompanying drawings wherein:
Figure 1 is an exploded perspective view of a first embodiment of the valve according to the invention;
Figure 2 is a perspective view from below of the flow control element of the valve shown in Figure 1;
Figure 3 is a cross-sectional view, taken along the line III-III, of the valve shown in Figure 1;
Figure 4 is a cross-sectional view of the valve shown in the previous figure, assembled;
Figure 5 is a cross-sectional view of the assembled valve shown in cross-section in the previous figure, taken along the line V-V;
Figures 6a to 6h are transverse cross-sectional views of the first embodiment of the valve according to the invention in eight possible mutually consecutive configurations;
Figure 7 is an exploded perspective view of a second embodiment of the valve;
Figure 8 is a perspective view from below of the flow control element of the valve shown in Figure 7;
Figure 9 is a transverse cross-sectional view of the valve shown in Figure 7, assembled, taken proximate to the bottom of the end of the flow control element;
Figures 10a to 10c are transverse cross-sectional views of the second embodiment of the valve according to the invention in three possible mutually consecutive configurations.

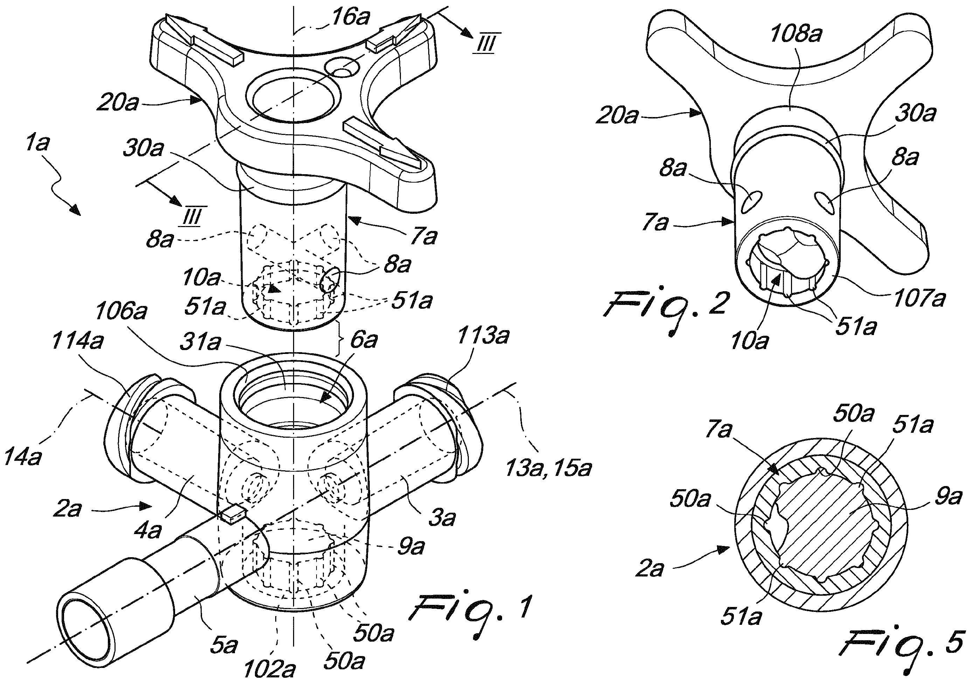

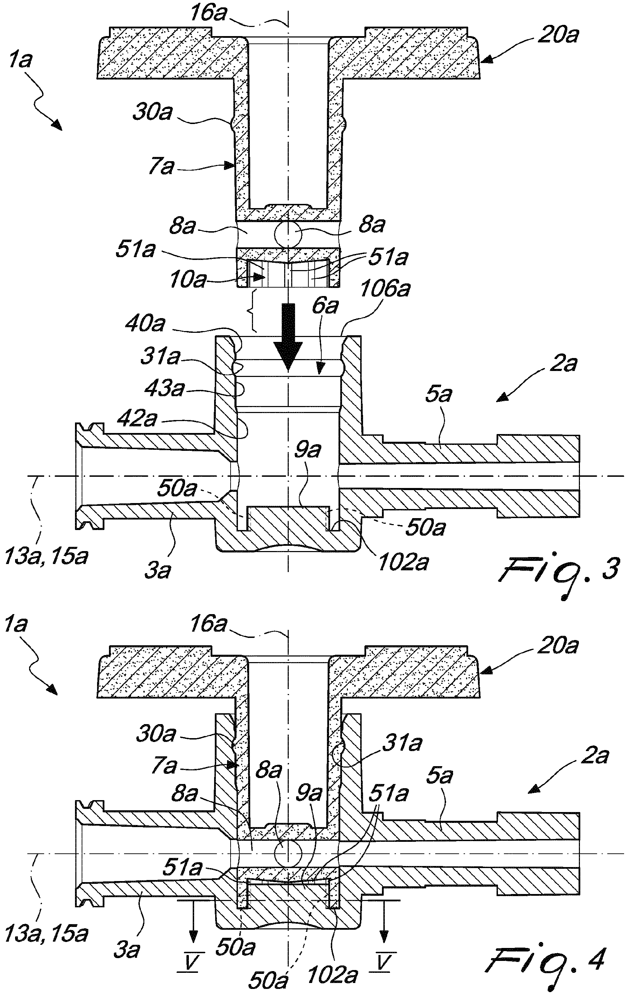

With reference to Figures 1 to 6h, the improved valve, particularly of the type for medical use, generally designated in the first embodiment with the reference numeral 1a, comprises a valve body 2a which has:
- at least one first inlet 3a oriented substantially along a first axis 13a that passes through the valve body 2a and shaped to be connected to at least one first delivery pipe of a first fluid;
- at least one outlet 5a oriented substantially along a second axis 15a that passes through the valve body 2a substantially incident to the first axis 13a and shaped to be connected to either at least one drainage pipe or at least one second valve;
- a chamber 6a defined at the intersection of the axes 13a and 15a in such a manner as to be passed through by them and be connected to the first inlet 3a and with the outlet 5a.

At least one flow control element 7a is further comprised, which is accommodated so that it can rotate inside the chamber 6a and has, on its outer surface, a plurality of ports 8a which are mutually connected.

The flow control element 7a is movable between at least one closed position, in which the chamber 6a is hydraulically isolated from either or both of the first inlet 3a and the outlet 5a, and at least one operative position, in which the first inlet 3a and the outlet 5a are mutually connected through the ports 8a.

According to the invention, the valve 1a comprises:
- a contoured protuberance 9a defined on either the bottom 102a of the chamber 6a or a first portion 107a of the flow control element 7a, which is designed to face toward such bottom 102a;
- a contoured recess 10a defined on the other one of either the bottom 102a or the first portion 107a.

Advantageously, the contoured protuberance 9a and the contoured recess 10a are geometrically shaped so as to define at least two mechanical click-stops for the relative rotation of the flow control element 7a with respect to the chamber 6a which correspond, respectively, to the closed position and to the operative position.

The expression "mechanical click-stops" means relative positions between the flow control element 7a and the chamber 6a that are stable and which define at least two configurations of use of the valve 1a.

As will be better described later, in this first embodiment, the contoured protuberance 9a is defined on the bottom of the chamber 6a, while the contoured recess 10a is defined on the first portion 107a of outer surface of the flow control element 7a which is designed to face toward the bottom 102a of the chamber 6a.

Advantageously, the valve 1a comprises activator means 20a of the flow control element 7a, which are directly associated therewith at a second portion 108a thereof that is external with respect to the chamber 6a and arranged opposite with respect to the first portion 107a.

Advantageously, such activator means 108a comprise a control knob with an ergonomic shape that can be manipulated manually by an operator, for example, by means of the use of two fingers.

Conveniently, the chamber 6a is substantially frustum-shaped with a blind circular base, extending along a third axis 16a which is incident to the first axis 13a and to the second axis 15a.

More specifically, the bottom 102a of the chamber 6a is defined at the minor base of the substantial frustum shape with a blind circular base.

Such a shape of the chamber 6a makes it possible to manufacture the valve body 2a using the direct injection technique, a process that produces substantially no waste material.

Advantageously, the flow control element 7a has a geometry that is substantially complementary to the substantial frustum shape with a blind circular base of the chamber 6a, so as to be accommodated inside such chamber 6a and creating a hydraulic seal with its walls.

In fact, as will be better described later, the flow control element 7a and the chamber 6a have particular geometric contrivances that create a hydraulic seal between them.

Conveniently, furthermore, the valve 1a comprises extraction-preventing means 30a and 31a of the flow control element 7a with respect to the chamber 6a along the third axis 16a.

In fact, the chamber 6a has an aperture 106a arranged opposite to the bottom 102a, for inserting the flow control element 7a into the chamber 6a along the third axis 16a.

Such extraction-preventing means 30a and 31a are defined partially by the flow control element 7a and partially by the chamber 6a.

Advantageously, such extraction-preventing means 30a and 31a comprise at least one annular ridge 30a defined on either the inner surface of the chamber 6a or the outer surface of the flow control element 7a, on a plane perpendicular to the third axis 16a, and a respective annular seat 31a defined on the other one of either the inner surface of the chamber 6a or the outer surface of the flow control element 7a.

The annular ridge 30a has a blended outer surface in order to allow the forced insertion of the flow control element 7a into the chamber 6a.

In the specific case of the first embodiment described here, the annular ridge 30a extends circumferentially on the outer surface of the flow control element 7a, on a plane perpendicular to the third axis 16a.

More specifically, such annular ridge 30a is defined, along the third axis 16a, on a portion of outer surface of the flow control element 7a comprised between the second portion 108a and the part of the chamber 6a that is connected to the first inlet 3a and the outlet 5a.

The annular seat 31a by contrast is defined on the inner walls of the chamber 6a, and has a geometry substantially complementary to the geometry that defines the annular ridge 30a.

In this manner, as a result of the insertion of the flow control element 7a into the chamber 6a, an extraction-preventing joint is created that makes it possible to manipulate the valve 1a without the risk of the flow control element 7a slipping out and exiting the chamber 6a in which it is accommodated.

Advantageously, the chamber 6a comprises a convergent section 40a that extends along the third axis 16a from the aperture 106a to the extraction-preventing means 30a and 31a, and the aperture 106a has an inner edge that is beveled for the facilitated insertion of the flow control element 7a into the chamber 6a.

Such chamber 6a further comprises an end section 42a, which extends at the first inlet 3a and at the outlet 5a and is radially narrower than the convergent section 40a and shaped so as to allow the forced accommodation of part of the flow control element 7a.

In this way a hydraulic seal is created between the inner walls of the chamber 6a and the outer walls of the flow control element 7a.

Furthermore, more specifically, between the sections 40a and 42a described above a straight section 43a is provided which is radially widened with respect to the end section 42a and is adapted to define a variation in transverse cross-section that is such as to contribute to the hydraulic seal between the chamber 6a and the flow control element 7a.

Advantageously, the valve 1a further comprises at least one second inlet 4a, oriented substantially along a fourth axis 14a that passes through the valve body 2a substantially incident to the first axis 13a, to the second axis 15a and to the third axis 16a, and which is adapted to be connected to a second delivery pipe of a second fluid.

Conveniently, the first axis 13a and the second axis 15a are substantially coincident.

Furthermore, the fourth axis 14a is substantially perpendicular to the first axis 13a and to the second axis 15a, and the third axis 16a is substantially perpendicular to the plane defined by the first axis 13a, by the second axis 15a and by the fourth axis 14a.

The number of ports 8a in the flow control element 7a is conveniently equal to the overall number of inlets and outlets on the valve body 2a; therefore, in the case considered here, the flow control element 7a has three ports 8a, respectively a first, a second and a third port.

The three ports 8a extend radially, substantially from the outer surface of the flow control element 7 with respect to the third axis 16a, where they are substantially incident.

The first port and the second port are substantially coaxial and are oriented along two parallel and coincident axes that substantially intersect the third axis 16a.

The third port is instead oriented along an axis that is substantially perpendicular, both to the two parallel and coincident axes and also to the third axis 16a or 16b.

The three ports extend substantially on a plane perpendicular to the third axis 16a, which substantially coincides with the plane that contains the first axis 13a, the second axis 15a and the fourth axis 14a.

In this manner, the axes on which the three ports are oriented are alignable respectively with the first axis 13a, the second axis 15a and the fourth axis 14a, following a relative rotation between the flow control element 7a and the chamber 6a.

The control knob is shaped like a "T" and has a substantially flat extension on a plane substantially perpendicular to the third axis 16a, reproducing the inner arrangement of channels of the ports 8a.

Furthermore, the first inlet 3a and the second inlet 4a respectively have a first threaded connector 113a and a second threaded connector 114a, which are adapted to be engaged respectively by a first threaded coupling and by a second threaded coupling, at the end of the first and/or of the second delivery pipe.

The outlet 5a on the other hand comprises a third connector 115a with a substantially frustum-shaped geometry adapted to be engaged by a third coupling at the end of the drainage pipe or of the second valve.

More specifically, such third connector 115a is a Luer cone.

Advantageously, either or both of the flow control element 7a and the valve body 2a is made of a softer and more elastically yielding material than the material with which the other one of either the flow control element 7a or the valve body 2a is made.

More specifically, the valve body 2a is made of polycarbonate and the flow control element 7a is made of polyolefin material with a lower hardness than polycarbonate, for example high-density polyethylene (HDPE).

Conveniently, furthermore, the valve 1a comprises a layer of silicone material interposed between the valve body 2a and the flow control element 7a, which is adapted to lubricate the flow control element 7a and to allow its relative rotation with respect to the valve body 6a.

The layer of silicone material is deposited, at the discretion of the assembly technician, on the inner surface of the chamber 6a and/or on the outer surface of the flow control element 7a, before the latter is inserted into the chamber 6a.

With reference to the first embodiment of the valve 1a, the geometry of the contoured protuberance 9a advantageously is substantially circular around the third axis 16a, with at least two semi-cylindrical lateral protuberances 50a aligned parallel with the third axis 16a.

Conveniently, the geometry of the contoured recess 10a is complementary to the contoured protuberance 9a, with at least two lateral seats 51a which correspond to such at least two lateral protuberances 50a.

Furthermore, the coupling of such at least two lateral protuberances 50a with such at least two lateral seats 51a defines the above-mentioned at least two mechanical click-stops.

In this, not least by virtue of the chosen materials, the two mechanical click-stops are elastically yielding so as to permit forced relative rotations of the contoured protuberance 9a in the contoured recess 10a and, at the same time, the exact positioning of the flow control element 7a in at least one from among such at least one closed position and such at least one operative position, as a result of the elastic yielding of either or both of the contoured protuberance 9a and the contoured recess 10a.

Advantageously, a plurality of lateral protuberances 50a is provided around the contoured protuberance 9a according to a circular array at the same constant pitch, so as to define a plurality of mechanical click-stops with constant pitch.

Furthermore, such at least one operative position therefore comprises a plurality of operative positions which correspond to at least some of the plurality of mechanical click-stops with constant pitch.

More specifically, the second lateral protuberances 50a extend on the outer side of the contoured protuberance 9a according to a semi-cylindrical geometric shape with an axis of longitudinal extension substantially parallel to the third axis 16a.

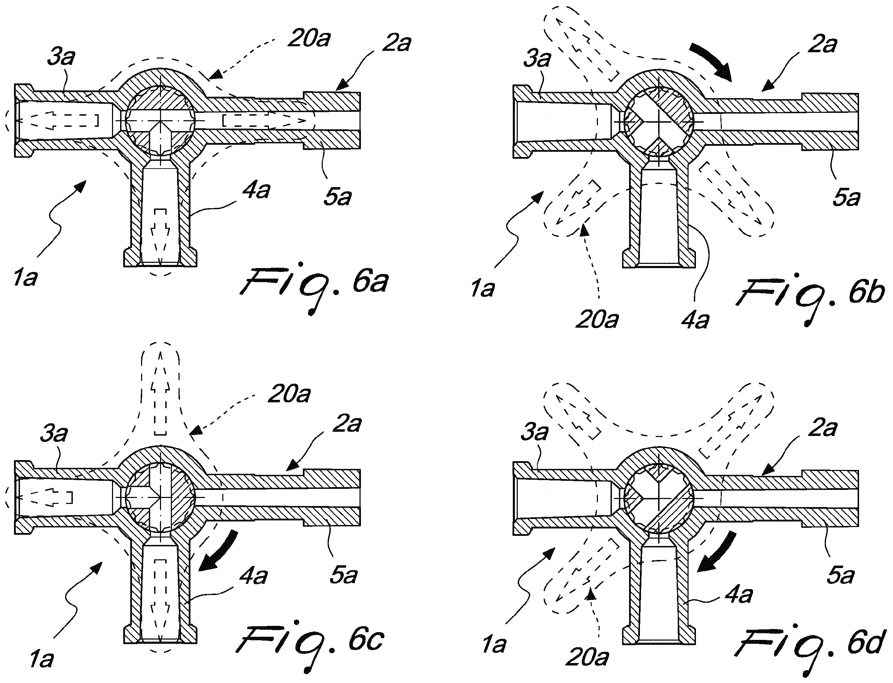

In the first embodiment, also, the contoured protuberance 9a has eight lateral protuberances 50a and the contoured recess 10a has eight corresponding lateral seats 51a, so as to define eight relative positions between the flow control element 7a and the valve body 2a, each one defined by a partial rotation of the flow control element 7a substantially equal to 45°, as shown in Figures 6a to 6h.

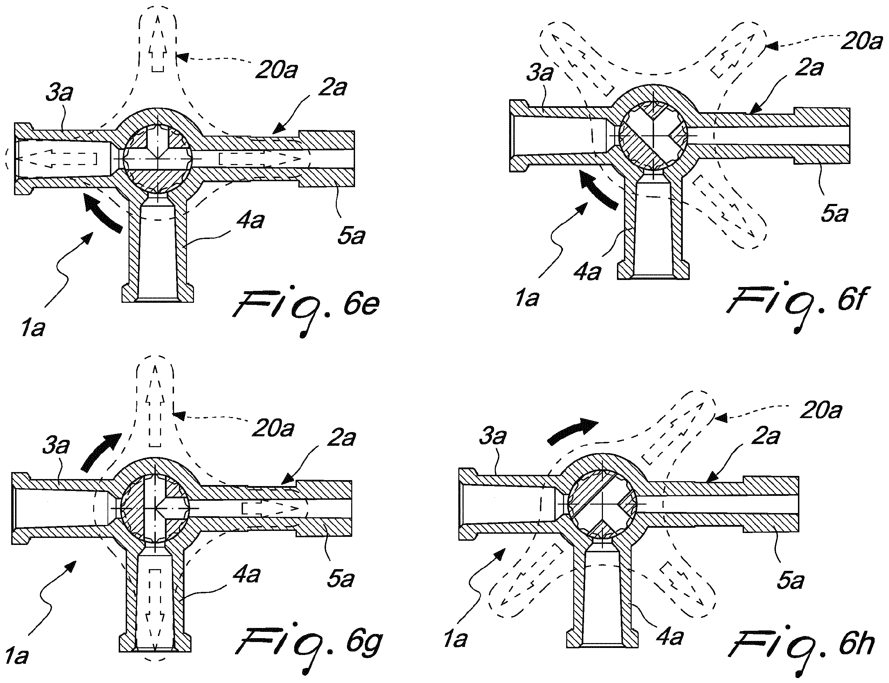

Advantageously, the plurality of operative positions comprises:
- a first operative position, in which the first inlet 3a, the second inlet 4a and the outlet 5a are hydraulically connected to the chamber 6a, as shown in Figure 6a;
- a second operative position, in which the first inlet 3a and the outlet 5a are hydraulically connected to the chamber 6a, while the second inlet 4a is hydraulically isolated, as shown in Figure 6e; and
- a third operative position, in which the second inlet 4a and the outlet 5a are hydraulically connected to the chamber 6a, while the first inlet 3a is hydraulically isolated, as shown in Figure 6g.

Conveniently, furthermore, such at least one closed position comprises:
- a first closed position, in which the first inlet 3a and the second inlet 4a are hydraulically connected to the chamber 6a, while the outlet 5a is hydraulically isolated, as shown in Figure 6c; and
- a plurality of second closed positions, corresponding to intermediate positions between such operative positions and such first closed position, in which the first inlet 3a, the second inlet 4a and the outlet 5a are hydraulically isolated, as shown respectively in Figures 6b, 6d, 6f and 6h.

In this way, the valve 1a can be used as a three-way valve.

Differently, if one of the inlets 3a and 4a should be used as an outlet, even temporarily, for the flow of fluid originating from the inlet, then the "first closed position" would take on the functionality of a "fourth operative position".

In this way, the valve 1a can be used as a four-way valve.

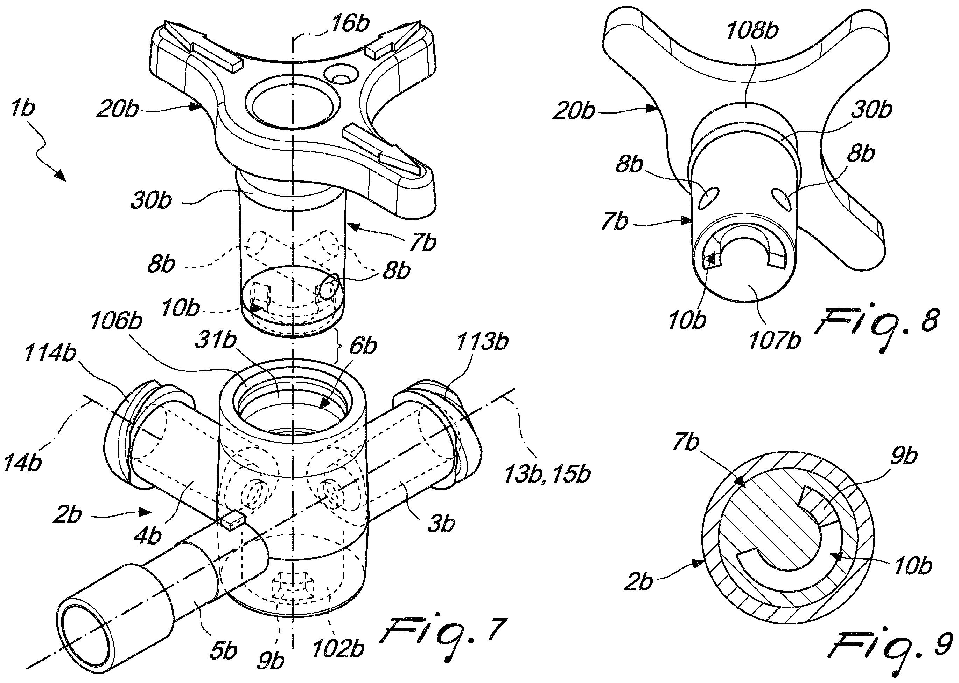

With reference to Figures 7 to 10c, the improved valve, particularly of the type for medical use, is generally designated in the second embodiment with the reference numeral 1b.

Such second embodiment has parts, elements and characteristics in common with or analogous to the first embodiment described above.

For this reason, in the figures cited above, these common or analogous parts, elements and characteristics, if shown, are indicated with the same reference numbers used for the first embodiment, replacing the subscript "a" with the subscript "b".

Differently from the first embodiment, the contoured recess 10b instead has a geometric shape that is substantially an arc of an annulus and the contoured protuberance 9b is accommodated therein with play so that it allows relative rotations between the flow control element 7b and the chamber 6b that are equal to the difference between the extent of the arc of an annulus and the thickness of the contoured protuberance 9b along the average circumference of said arc of an annulus.

More specifically, in the valve 1b shown in Figure 7, the geometry of the contoured protuberance 9b is also that of an arc of an annulus, of lesser extent than the extent of the arc of an annulus that defines the geometric shape of the recess 10b.

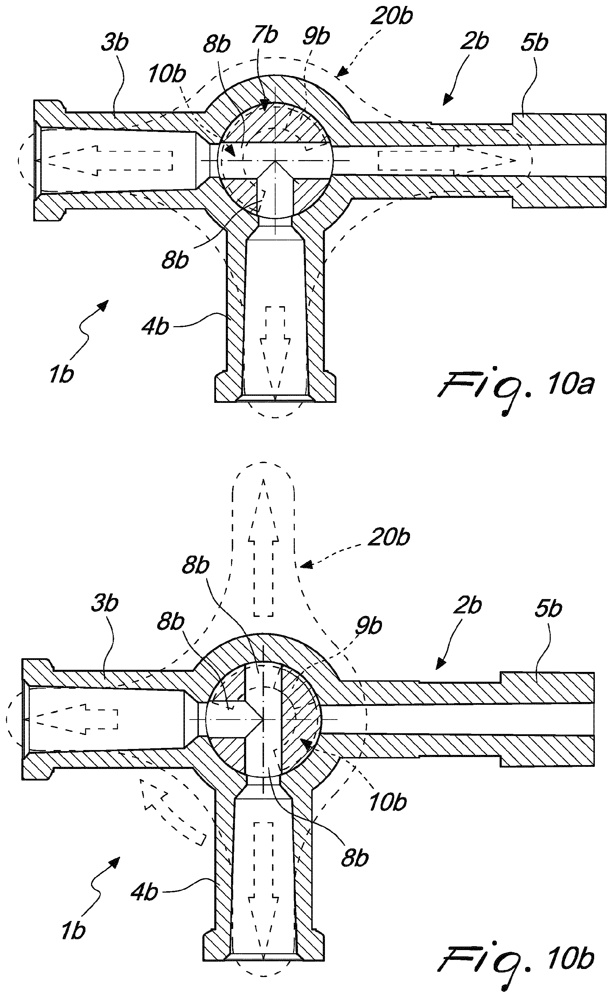

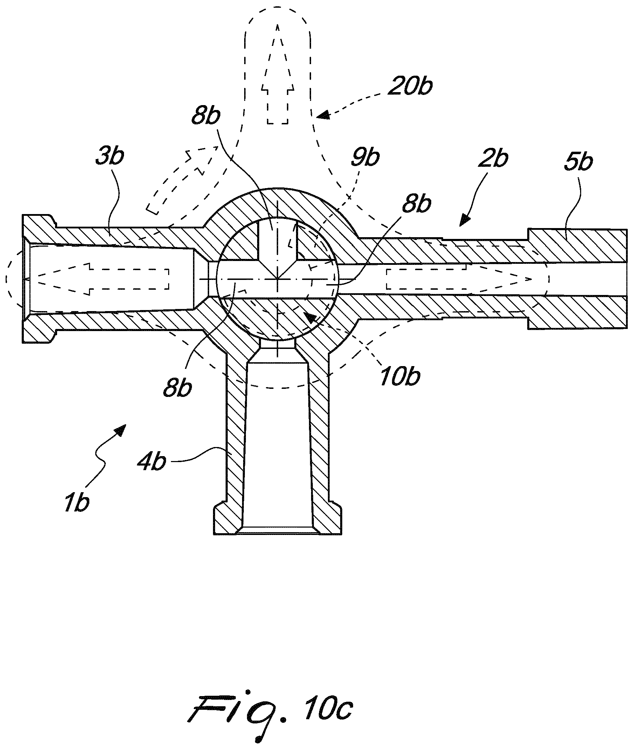

In this way, it is possible to select the degrees of rotation of the flow control element 7b, so as to have a rotation limited to 90°, as shown in Figures 10a to 10b, so providing a two-way valve, or a rotation limited to 180°, as shown in Figures 10a to 10c, so providing a three-way valve.

The operation of the valve 1a or 1b is clear and easily intuited from the foregoing description.

More specifically, relative rotations between the flow control element 7a or 7b and the valve body 2a or 2b make it possible to define different configurations of use of the valve 1a or 1b, which permit and/or stop the passage of a fluid through the inlets and/or the outlet of the valve body 2a or 2b, so regulating the direction of its flow.

In practice it has been found that the improved valve, particularly of the type for medical use, according to the present invention achieves the set aim and objects, in that it makes it possible to regulate the passage of one or more fluids while preventing leaks in any configuration of use, by virtue of the continuity of the fluid-tight walls between the flow control element and the internal chamber.

In fact, the particular shape of the bottom of the chamber and of the flow control element means the above-mentioned fluid-tight walls are not subject to deformations during the rotation of the flow control element inside the chamber.

Another advantage of the valve according to the present invention consists in that it can be easily used and it is simple in terms of construction.

Another advantage of the valve according to the invention consists in that it is made with mechanical machining processes that substantially do not produce waste material.

Another advantage of the valve according to the invention consists in that, by virtue of the chamber with a closed bottom, it further limits the possibility of leaks.

The valve, thus conceived, is susceptible of numerous modifications and variations, all of which are within the scope of the appended claims.

For example, the inlets and the outlet described here can have different shape structures according to the coupling required with the delivery and/or drainage pipes.

Furthermore, the functionality of said inlets/outlet can be inverted, and the latter can act respectively as outlets/inlet according to requirements.

Moreover, all the details may be substituted by other, technically equivalent elements.

In practice the materials employed, provided they are compatible with the specific use, and the contingent dimensions and shapes, may be any according to requirements and to the state of the art.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. An improved valve (1a, 1b), particularly of the type for medical use, comprising a valve body (2a, 2b) that has:
- at least one first inlet (3a, 3b) oriented substantially along a first axis (13a, 13b) that passes through said valve body (2a, 2b) and shaped to be connected to at least one first delivery pipe of a first fluid;
- at least one outlet (5a, 5b) oriented substantially along a second axis (15a, 15b) that passes through said valve body (2a, 2b) substantially incident to said first axis (13a, 13b) and shaped to be connected to either at least one drainage pipe or at least one second valve;
- a chamber (6a, 6b) defined at the intersection of said axes (13a, 13b, 15a, 15b) in such a manner as to be passed through by said axes (13a, 13b, 15a, 15b) and be connected to said at least one first inlet (3a, 3b) and with said at least one outlet (5a, 5b);
at least one flow control element (7a, 7b) being further comprised which is accommodated so that it can rotate inside said chamber (6a, 6b) and has, on its outer surface, a plurality of ports (8a, 8b) which are mutually connected;
said flow control element (7a, 7b) being movable between at least one closed position, in which said chamber (6a, 6b) is hydraulically isolated from either or both of said at least one first inlet (3a, 3b) and said at least one outlet (5a, 5b), and at least one operative position, in which said at least one first inlet (3a, 3b) and said at least one outlet (5a, 5b) are mutually connected through said ports (8a, 8b);
wherein it comprises:
- a contoured protuberance (9a, 9b) defined on either the bottom (102a, 102b) of said chamber (6a, 6b) or a first portion (107a, 107b) of said flow control element (7a, 7b), which is designed to face toward said bottom (102a, 102b);
- a contoured recess (10a, 10b) defined on the other one of either said bottom (102a, 102b) or said first portion (107a, 107b);
said contoured protuberance (9a, 9b) and said contoured recess (10a, 10b) being geometrically shaped so as to define at least two mechanical click-stops for the relative rotation of said flow control element (7a, 7b) with respect to said chamber (6a, 6b) which correspond, respectively, to said at least one closed position and to said at least one operative position, **characterized in that** it further comprises a layer of silicone material interposed between said valve body (2a, 2b) and said flow control element (7a, 7b), which is adapted to lubricate said flow control element (7a, 7b) and is adapted to allow the relative rotation of said flow control element (7a, 7b) with respect to said valve body (2a, 2b).

2. The valve (1a, 1b) according to claim 1, **characterized in that** said valve (1a, 1b) comprises activator means (20a, 20b) of said flow control element (7a, 7b) which are directly associated with said flow control element (7a, 7b) at a second portion (108a, 108b) of said flow control element (7a, 7b) that is external with respect to said chamber (6a, 6b) and arranged opposite with respect to said first portion (107a, 107b).

3. The valve (1a, 1b) according to claims 1 or 2, **characterized in that** said chamber (6a, 6b) is substantially frustum-shaped with a blind circular base extending along a third axis (16a, 16b) which is incident to said first axis and said second axis (13a, 13b, 15a, 15b); the bottom (102a, 102b) of said chamber (6a, 6b) being defined at the minor base of said substantial frustum shape with a blind circular base.

4. The valve (1a, 1b) according to claim 3, **characterized in that** said flow control element (7a, 7b) has a geometry substantially complementary to said substantial frustum shape with a blind circular base of said chamber (6a, 6b).

5. The valve (1a, 1b) according to claim 4, **characterized in that** it comprises extraction-preventing means (30a, 31a, 30b, 31b) of said flow control element (7a, 7b) with respect to said chamber (6a, 6b) along said third axis (16a, 16b) and defined partially by said flow control element (7a, 7b) and partially by said chamber (6a, 6b); said chamber (6a, 6b) has an aperture (106a, 106b) arranged opposite to said bottom (102a, 102b) for the insertion of said flow control element (7a, 7b) into said chamber (6a, 6b) along said third axis (16a, 16b).

6. The valve (1a, 1b) according to claim 5, **characterized in that** said extraction-preventing means comprise at least one annular ridge (30a, 30b) defined on either the inner surface of said chamber (6a, 6b) or the outer surface of said flow control element (7a, 7b), on a plane perpendicular to said third axis (16a, 16b), and a respective annular seat (31a, 31b) defined on the other one of either said inner surface of said chamber (6a, 6b) or said outer surface of said flow control element (7a, 7b); said annular ridge (30a, 30b) has a blended surface in order to allow the forced insertion of said flow control element (7a, 7b) into said camera (6a, 6b).

7. The valve (1a, 1b) according to claim 6, **characterized in that** said chamber (6a, 6b) comprises a convergent section (40a), extending along said third axis (16a, 16b) from said aperture (106a, 106b) to said extraction-preventing means (30a, 31a, 30b, 31b) and **in that** said aperture (106a, 106b) has an inner edge that is beveled for the facilitated insertion of said flow control element (7a, 7b) into said chamber (6a, 6b); said chamber (6a, 6b) further comprises an end section (42a), which extends at said at least one first inlet (3a, 3b) and at said at least one outlet (5a, 5b) and is radially narrower than said convergent section (40a) and shaped so as to allow the forced accommodation of part of said flow control element (7a, 7b).

8. The valve (1a, 1b) according to one or more of claims 1 to 7, **characterized in that** it comprises at least one second inlet (4a, 4b), oriented substantially along a fourth axis (14a, 14b) that passes through said valve body (2a, 2b) substantially incident to said first axis (13a, 13b), to said second axis (15a, 15b) and to said third axis (16a, 16b), and is adapted to be connected to a second delivery pipe of a second fluid.

9. The valve (1a, 1b) according to claim 8, **characterized in that**:
said first axis (13a, 13b) and said second axis (15a, 15b) are substantially coincident,
said fourth axis (14a, 14b) is substantially at right angles to said first axis (13a, 13b) and to said second axis (15a, 15b) and
said third axis (16a, 16b) is substantially perpendicular to the plane defined by said first axis (13a, 13b), said second axis (15a, 15b) and said fourth axis (14a, 14b).

10. The valve (1a, 1b) according to one or more of claims 1 to 9, **characterized in that** either or both of said flow control element (7a, 7b) and said valve body (2a, 2b) is made of a softer and more elastically yielding material than the material with which the other one of either said flow control element (7a, 7b) or said valve body (2a, 2b) is made.

11. The valve (1a) according to one or more of claims 1 to 10, **characterized in that** said contoured protuberance (9a) has a substantially circular geometry around said third axis (16a) with at least two semi-cylindrical lateral protuberances (50a) aligned parallel with said third axis (16a) and **in that** the geometry of said contoured recess (10a) is complementary to said contoured protuberance (9a) with at least two lateral seats (51a) which correspond to said at least two lateral protuberances (50a); the coupling of said at least two lateral protuberances (50a) with said at least two lateral seats (51a) defines said at least two mechanical click-stops.

12. The valve (1a) according to claim 1, **characterized in that** said at least two lateral protuberances (50a) comprise a plurality of lateral protuberances (50a) arranged around said contoured protuberance (9a) in a circular array with constant pitch so as to define a plurality of mechanical click-stops with constant pitch; said at least one operative position comprising a plurality of operative positions of said flow control element (7a) which correspond to at least part of said plurality of mechanical click-stops with constant pitch.

13. The valve (1a) according to claim 12, **characterized in that** said plurality of operative positions comprise:
- a first operative position, in which said at least one first inlet (3a), said at least one second inlet (4a) and said at least one outlet (5a) are hydraulically connected to said chamber (6a);
- a second operative position, in which said at least one first inlet (3a) and said at least one outlet (5a) are hydraulically connected to said chamber (6a), while said at least one second inlet (4a) is hydraulically isolated; and
- a third operative position, in which said at least one second inlet (4a) and said at least one outlet (5a) are hydraulically connected to said chamber (6a), while said at least one first inlet (3a) is hydraulically isolated;
and **in that** said at least one closed position comprises:
- a first closed position, in which said at least one first inlet (3a) and said at least one second inlet (4a) are hydraulically connected to said chamber (6a), while said at least one outlet (5a) is hydraulically isolated; and
- a plurality of second closed positions, corresponding to intermediate positions between said operative positions and said first closed position, in which said at least one first inlet (3a), said at least one second inlet (4a) and at least one outlet (5a) are hydraulically isolated.

14. The valve (1b) according to one or more of claims 1 to 11, **characterized in that** said contoured recess (10b) has a geometric shape that is substantially an arc of an annulus and **in that** said contoured protuberance (9b) is accommodated with play in said contoured recess (10b) so that it allows relative rotations between said flow control element (7b) and said chamber (6b) that are equal to the difference between the extent of said arc of an annulus and the thickness of said contoured protuberance (9b) along the average circumference of said arc of an annulus.

## Patentansprüche

1. Ein verbessertes Ventil (1a, 1b), insbesondere von der Art für medizinische Zwecke, das einen Ventilkörper (2a, 2b) umfasst, der Folgendes hat:
- mindestens einen ersten Einlass (3a, 3b), ausgerichtet im Wesentlichen entlang einer ersten Achse (13a, 13b), die durch den Ventilkörper (2a, 2b) verläuft, und geformt, um mit mindestens einer ersten Förderleitung für ein erstes Fluid verbunden zu werden;
- mindestens einen Auslass (5a, 5b), ausgerichtet im Wesentlichen entlang einer zweiten Achse (15a, 15b), die durch den Ventilkörper (2a, 2b) verläuft, im Wesentlichen einfallend mit der ersten Achse (13a, 13b) und geformt, um entweder mit mindestens einer Ablassleitung oder mindestens einem zweiten Ventil verbunden zu werden;
- eine Kammer (6a, 6b), bestimmt am Schnittpunkt der Achsen (13a, 13b, 15a, 15b) derart, dass sie von den Achsen (13a, 13b, 15a, 15b) durchquert und mit dem mindestens einen ersten Einlass (3a, 3b) und mit dem mindestens einen Auslass (5a, 5b) verbunden wird;
wobei weiter mindestens ein Durchflussregelelement (7a, 7b) umfasst ist, welches drehbar in die Kammer (6a, 6b) aufgenommen ist und an seiner Außenfläche eine Vielzahl miteinander verbundener Öffnungen (8a, 8b) hat;
wobei das Durchflussregelelement (7a, 7b) beweglich ist zwischen mindestens einer geschlossenen Position, in welcher die Kammer (6a, 6b) gegenüber dem mindestens einen ersten Einlass (3a, 3b) und/oder dem mindestens einen Auslass (5a, 5b) hydraulisch isoliert ist, und mindestens einer Arbeitsposition, in welcher der mindestens eine erste Einlass (3a, 3b) und der mindestens eine Auslass (5a, 5b) miteinander über die Öffnungen (8a, 8b) verbunden sind;
wobei es Folgendes umfasst:
- einen konturierten Vorsprung (9a, 9b), bestimmt entweder am Boden (102a, 102b) der Kammer (6a, 6b) oder einem ersten Abschnitt (107a, 107b) des Durchflussregelelements (7a, 7b), der dazu bestimmt ist, dem Boden (102a, 102b) zugewandt zu sein;
- eine konturierte Vertiefung (10a, 10b), bestimmt an dem jeweils anderen entweder des Bodens (102a, 102b) oder des ersten Abschnitts (107a, 107b);
wobei der konturierte Vorsprung (9a, 9b) und die konturierte Vertiefung (10a, 10b) geometrisch geformt sind, um mindestens zwei mechanische Klickrastungen für die Relativdrehung des Durchflussregelelements (7a, 7b) mit Bezug auf die Kammer (6a, 6b) zu bestimmen, welche der mindestens einen geschlossenen Position beziehungsweise der mindestens einen Arbeitsposition entsprechen; **dadurch gekennzeichnet, dass** es weiter eine Schicht aus Silikonmaterial umfasst, angeordnet zwischen dem Ventilkörper (2a, 2b) und dem Durchflussregelelement (7a, 7b), die ausgebildet ist, um das Durchflussregelelement (7a, 7b) zu schmieren und um die Relativdrehung des Durchflussregelelements (7a, 7b) mit Bezug auf den Ventilkörper (2a, 2b) zu gestatten.

2. Das Ventil (1a, 1b) gemäß Anspruch 1, **dadurch gekennzeichnet, dass** das Ventil (1a, 1b) Aktivierungsmittel (20a, 20b) für das Durchflussregelelement (7a, 7b) umfasst, die an einem zweiten Abschnitt (108a, 108b) des Durchflussregelelements (7a, 7b), der außerhalb der Kammer (6a, 6b) liegt und gegenüber dem ersten Abschnitt (107a, 107b) angeordnet ist, direkt mit dem Durchflussregelelement (7a, 7b) verbunden sind.

3. Das Ventil (1a, 1b) gemäß Anspruch 1 oder 2, **dadurch gekennzeichnet, dass** die Kammer (6a, 6b) im Wesentlichen stumpfförmig ist, mit einer blinden kreisförmigen Basis, die sich entlang einer dritten Achse (16a, 16b) erstreckt, welche mit der ersten Achse und der zweiten Achse (13a, 13b, 15a, 15b) einfällt*,* wobei der Boden (102a, 102b) der Kammer (6a, 6b) an der kleineren Basis der im Wesentlichen Stumpf-Form mit blinder kreisförmiger Basis bestimmt ist.

4. Das Ventil (1a, 1b) gemäß Anspruch 3, **dadurch gekennzeichnet, dass** das Durchflussregelelement (7a, 7b) eine Form hat, die im Wesentlichen komplementär zu der im Wesentlichen Stumpf-Form mit blinder kreisförmiger Basis der Kammer (6a, 6b) ist.

5. Das Ventil (1a, 1b) gemäß Anspruch 4, **dadurch gekennzeichnet, dass** es extraktionsverhindernde Mittel (30a, 31a, 30b, 31b) des Durchflussregelelements (7a, 7b) mit Bezug auf die Kammer (6a, 6b) entlang der dritten Achse (16a, 16b) und bestimmt teilweise durch das Durchflussregelelement (7a, 7b) und teilweise durch die Kammer (6a, 6b) umfasst; wobei die Kammer (6a, 6b) eine Öffnung (106a, 106b) hat, angeordnet gegenüber dem Boden (102a, 102b) zum Einsetzen des Durchflussregelelements (7a, 7b) in die Kammer (6a, 6b) entlang der dritten Achse (16a, 16b).

6. Das Ventil (1a, 1b) gemäß Anspruch 5, **dadurch gekennzeichnet, dass** die extraktionsverhindernden Mittel mindestens einen ringförmigen Grat (30a, 30b) umfassen, bestimmt entweder an der Innenfläche der Kammer (6a, 6b) oder an der Außenfläche des Durchflussregelelements (7a, 7b) auf einer Ebene senkrecht zu der dritten Achse (16a, 16b), und einen entsprechenden ringförmigen Sitz (31a, 31b), bestimmt an dem jeweils anderen entweder der Innenfläche der Kammer (6a, 6b) oder an der Außenfläche des Durchflussregelelements (7a, 7b); der ringförmige Grat (30a, 30b) hat eine Anschlussoberfläche, um das Hineinpressen des Durchflussregelelements (7a, 7b) in die Kammer (6a, 6b) zu ermöglichen.

7. Das Ventil (1a, 1b) gemäß Anspruch 6, **dadurch gekennzeichnet, dass** die Kammer (6a, 6b) einen konvergierenden Abschnitt (40a) umfasst, der sich entlang der dritten Achse (16a, 16b) von der Öffnung (106a, 106b) zu den extraktionsverhindernden Mitteln (30a, 31a, 30b, 31b) erstreckt, und dadurch, dass die Öffnung (106a, 106b) eine Innenkante hat, die zur Vereinfachung des Einsetzens des Durchflussregelelements (7a, 7b) in die Kammer (6a, 6b) abgeschrägt ist; die Kammer (6a, 6b) umfasst weiter einen Endabschnitt (42a), der sich an dem mindestens einen ersten Einlass (3a, 3b) und an dem mindestens einen Auslass (5a, 5b) erstreckt und radial schmaler als der konvergierende Abschnitt (40a) und geformt ist, um die erzwungene Aufnahme eines Teils des Durchflussregelelements (7a, 7b) zu ermöglichen.

8. Das Ventil (1a, 1b) gemäß einem oder mehreren der Ansprüche 1 bis 7, **dadurch gekennzeichnet, dass** es mindestens einen zweiten Einlass (4a, 4b) umfasst, ausgerichtet im Wesentlichen entlang einer vierten Achse (14a, 14b), die durch den Ventilkörper (2a, 2b) verläuft, im Wesentlichen mit der ersten Achse (13a, 13b), mit der zweiten Achse (15a, 15b) und mit der dritten Achse (16a, 16b) einfallend; und ausgebildet ist, um mit einer zweiten Förderleitung für ein zweites Fluid verbunden zu werden.

9. Das Ventil (1a, 1b) gemäß Anspruch 8, **dadurch gekennzeichnet, dass**
die erste Achse (13a, 13b) und die zweite Achse (15a, 15b) im Wesentlichen zusammenfallen,
die vierte Achse (14a, 14b) im Wesentlichen in rechten Winkeln zu der ersten Achse (13a, 13b) und zu der zweiten Achse (15a, 15b) liegt und
die dritte Achse (16a, 16b) im Wesentlichen senkrecht zu der Ebene ist, die von der ersten Achse (13a, 13b), der zweiten Achse (15a, 15b) und der vierten Achse (14a, 14b) bestimmt ist.

10. Das Ventil (1a, 1b) gemäß einem oder mehreren der Ansprüche 1 bis 9, **dadurch gekennzeichnet, dass** das Durchflussregelelement (7a, 7b) und/oder der Ventilkörper (2a, 2b) aus einem Material bestehen, das weicher und elastisch nachgiebiger ist als das Material, aus dem das jeweils andere des Durchflussregelelements (7a, 7b) oder des Ventilkörpers (2a, 2b) besteht.

11. Das Ventil (1a) gemäß einem oder mehreren der Ansprüche 1 bis 10, **dadurch gekennzeichnet, dass** der konturierte Vorsprung (9a) eine im Wesentlichen kreisförmige Geometrie um die dritte Achse (16a) hat, mit mindestens zwei halbzylindrischen seitlichen Vorsprüngen (50a), ausgerichtet parallel zu der dritten Achse (16a), und dadurch, dass die Geometrie der konturierten Vertiefung (10a) komplementär zu dem konturierten Vorsprung (9a) ist, mit mindestens zwei seitlichen Sitzen (51a), die den mindestens zwei seitlichen Vorsprüngen (50a) entsprechen; wobei die Kopplung der mindestens zwei seitlichen Vorsprünge (50a) mit den mindestens zwei seitlichen Sitzen (51a) die mindestens zwei mechanischen Klickrastungen bestimmt.

12. Das Ventil (1a) gemäß Anspruch 1, **dadurch gekennzeichnet, dass** die mindestens zwei seitlichen Vorsprünge (50a) eine Vielzahl seitlicher Vorsprünge (50a) umfassen, die um den konturierten Vorsprung (9a) herum in einer kreisförmigen Anordnung mit konstanter Steigung angeordnet sind, um eine Vielzahl mechanischer Klickrastungen mit konstanter Steigung zu bestimmen; wobei die mindestens eine Arbeitsposition eine Vielzahl von Arbeitspositionen des Durchflussregelelements (7a) umfasst, die zumindest einem Teil der Vielzahl mechanischer Klickrastungen mit konstanter Steigung entsprechen.

13. Das Ventil (1a) gemäß Anspruch 12, **dadurch gekennzeichnet, dass** die Vielzahl von Arbeitspositionen Folgendes umfasst:
- eine erste Arbeitsposition, in welcher der mindestens eine erste Einlass (3a), der mindestens eine zweite Einlass (4a) und der mindestens eine Auslass (5a) hydraulisch mit der Kammer (6a) verbunden sind;
- eine zweite Arbeitsposition, in welcher der mindestens eine erste Einlass (3a) und der mindestens eine Auslass (5a) hydraulisch mit der Kammer (6a) verbunden sind, während der mindestens eine zweite Einlass (4a) hydraulisch isoliert ist; und
- eine dritte Arbeitsposition, in welcher der mindestens eine zweite Einlass (4a) und der mindestens eine Auslass (5a) hydraulisch mit der Kammer (6a) verbunden sind, während der mindestens eine erste Einlass (3a) hydraulisch isoliert ist;
und dadurch, dass die mindestens eine geschlossene Position Folgendes umfasst:
- eine erste geschlossene Position, in welcher der mindestens eine erste Einlass (3a) und der mindestens eine zweite Einlass (4a) hydraulisch mit der Kammer (6a) verbunden sind, während der mindestens eine Auslass (5a) hydraulisch isoliert ist; und
- eine Vielzahl zweiter geschlossener Positionen, die Zwischenpositionen zwischen den Arbeitspositionen und der ersten geschlossenen Position entsprechen und in denen der mindestens eine erste Einlass (3a), der mindestens eine zweite Einlass (4a) und mindestens ein Auslass (5a) hydraulisch isoliert sind.

14. Das Ventil (1b) gemäß einem oder mehreren der Ansprüche 1 bis 11, **dadurch gekennzeichnet, dass** die konturierte Vertiefung (10b) eine geometrische Form hat, die im Wesentlichen ein Bogen eines Kreisrings ist, und dadurch, dass der konturierte Vorsprung (9b) mit Spiel so in der konturierten Vertiefung (10b) untergebracht ist, dass er Relativdrehungen zwischen dem Durchflussregelelement (7b) und der Kammer (6b) gestattet, die gleich der Differenz zwischen der Ausdehnung des Bogens eines Kreisrings und der Dicke des konturierten Vorsprungs (9b) entlang dem durchschnittlichen Umfang des Bogens eines Kreisrings sind.

## Revendications

1. Valve améliorée (1a, 1b), notamment du type destiné à un usage médical, comprenant un corps de valve (2a, 2b) qui comporte :
- au moins une première entrée (3a, 3b) orientée sensiblement selon un premier axe (13a, 13b) qui traverse ledit corps de valve (2a, 2b) et conçue pour être raccordée à au moins un premier tuyau d'alimentation d'un premier fluide ;
- au moins une sortie (5a, 5b) orientée sensiblement le long d'un deuxième axe (15a, 15b) qui traverse ledit corps de valve (2a, 2b) de manière sensiblement incident audit premier axe (13a, 13b) et configurée pour être raccordée soit à au moins un tuyau de drainage, soit à au moins une deuxième valve ;
- une chambre (6a, 6b) définie à l'intersection desdits axes (13a, 13b, 15a, 15b) de manière à être traversée par lesdits axes (13a, 13b, 15a, 15b) et connectée à ladite au moins une première entrée (3a, 3b) et avec ladite au moins une sortie (5a, 5b) ;
au moins un élément de régulation de débit (7a, 7b) étant en outre prévu, lequel est logé de manière à pouvoir tourner à l'intérieur de ladite chambre (6a, 6b) et comporte, sur sa surface extérieure, une pluralité d'orifices (8a, 8b) qui sont reliés entre eux ;
ledit élément de régulation de débit (7a, 7b) étant mobile entre au moins une position fermée, dans laquelle ladite chambre (6a, 6b) est isolée hydrauliquement de l'une ou l'autre, ou des deux, de ladite au moins une première entrée (3a, 3b) et de ladite au moins une sortie (5a, 5b), et au moins une position de fonctionnement, dans laquelle ladite au moins une première entrée (3a, 3b) et ladite au moins une sortie (5a, 5b) sont reliées entre elles par l'intermédiaire desdits orifices (8a, 8b) ;
où elle comprend :
- une protubérance profilée (9a, 9b) définie soit sur le fond (102a, 102b) de ladite chambre (6a, 6b), soit sur une première partie (107a, 107b) dudit élément de régulation de débit (7a, 7b), qui est conçue pour faire face audit fond (102a, 102b) ;
- un évidement profilé (10a, 10b) défini sur l'autre parmi ledit fond (102a, 102b) ou ladite première partie (107a, 107b) ;
ladite protubérance profilée (9a, 9b) et ledit évidement profilé (10a, 10b) étant géométriquement conformés de manière à définir au moins deux crans mécaniques pour la rotation relative dudit élément de régulation de débit (7a, 7b) par rapport à ladite chambre (6a, 6b), qui correspondent respectivement à ladite au moins une position fermée et à ladite au moins une position de fonctionnement, **caractérisée en ce qu'**elle comprend en outre une couche de matériel en silicone intercalée entre ledit corps de valve (2a, 2b) et ledit élément de régulation de débit (7a, 7b), qui est adaptée pour lubrifier ledit élément de régulation de débit (7a, 7b) et est adaptée pour permettre la rotation relative dudit élément de régulation de débit (7a, 7b) par rapport audit corps de valve (2a, 2b).

2. La valve (1a, 1b) selon la revendication 1, **caractérisée en ce que** ladite valve (1a, 1b) comprend des moyens d'actionnement (20a, 20b) dudit élément de régulation de débit (7a, 7b) qui sont directement associés audit élément de régulation de débit (7a, 7b) au niveau d'une deuxième partie (108a, 108b) dudit élément de régulation de débit (7a, 7b) qui est externe par rapport à ladite chambre (6a, 6b) et disposée à l'opposé de ladite première partie (107a, 107b).

3. La valve (1a, 1b) selon les revendications 1 ou 2, **caractérisée en ce que** ladite chambre (6a, 6b) a sensiblement la forme d'un tronc de cône avec une base circulaire fermée s'étendant le long d'un troisième axe (16a, 16b) qui est incident audit premier axe et audit deuxième axe (13a, 13b, 15a, 15b) ; le fond (102a, 102b) de ladite chambre (6a, 6b) étant défini au niveau de la base mineure de ladite forme sensiblement en tronc de cône avec une base circulaire fermée.

4. La valve (la, lb) selon la revendication 3, **caractérisée en ce que** ledit élément de régulation de débit (7a, 7b) présente une géométrie sensiblement complémentaire à ladite forme sensiblement en tronc de cône à base circulaire fermée de ladite chambre (6a, 6b).

5. La valve (la, lb) selon la revendication 4, **caractérisée en ce qu'**elle comprend des moyens anti-extraction (30a, 31a, 30b, 31b) empêchant l'extraction dudit élément de régulation de débit (7a, 7b) par rapport à ladite chambre (6a, 6b) le long dudit troisième axe (16a, 16b) et définis en partie par ledit élément de régulation de débit (7a, 7b) et en partie par ladite chambre (6a, 6b) ; ladite chambre (6a, 6b) comporte une ouverture (106a, 106b) disposée à l'opposé dudit fond (102a, 102b) pour l'insertion dudit élément de régulation de débit (7a, 7b) dans ladite chambre (6a, 6b) le long dudit troisième axe (16a, 16b).

6. Valve (la, lb) selon la revendication 5, **caractérisée en ce que** lesdits moyens anti-extraction comprennent au moins une arête annulaire (30a, 30b) définie soit sur la surface intérieure de ladite chambre (6a, 6b), soit sur la surface extérieure dudit élément de régulation de débit (7a, 7b), dans un plan perpendiculaire audit troisième axe (16a, 16b), et un siège annulaire respectif (31a, 31b) défini sur l'autre desdites surface intérieure de ladite chambre (6a, 6b) ou surface extérieure dudit élément de régulation de débit (7a, 7b) ; ladite arête annulaire (30a, 30b) présente une surface raccordée afin de permettre l'insertion forcée dudit élément de régulation de débit (7a, 7b) dans ladite chambre (6a, 6b).

7. Valve (1a, 1b) selon la revendication 6, **caractérisée en ce que** ladite chambre (6a, 6b) comprend une section convergente (40a), s'étendant le long dudit troisième axe (16a, 16b) depuis ladite ouverture (106a, 106b) jusqu'auxdits moyens anti-extraction (30a, 31a, 30b, 31b) et **en ce que** ladite ouverture (106a, 106b) présente un bord intérieur chanfreiné pour faciliter l'insertion dudit élément de régulation de débit (7a, 7b) dans ladite chambre (6a, 6b) ; ladite chambre (6a, 6b) comprend en outre une section d'extrémité (42a), qui s'étend au niveau de ladite au moins une première entrée (3a, 3b) et au niveau de ladite au moins une sortie (5a, 5b) et qui est radialement plus étroite que ladite section convergente (40a) et formée de manière à permettre l'enclenchement forcé d'une partie dudit élément de régulation de débit (7a, 7b) .

8. Valve (1a, 1b) selon l'une ou plusieurs des revendications 1 à 7, **caractérisée en ce qu'**elle comprend au moins une deuxième entrée (4a, 4b), orientée sensiblement le long d'un quatrième axe (14a, 14b) qui traverse ledit corps de valve (2a, 2b) de manière sensiblement incident audit premier axe (13a, 13b), audit deuxième axe (15a, 15b) et audit troisième axe (16a, 16b), et est adaptée pour être raccordée à un deuxième tuyau d'alimentation d'un deuxième fluide.

9. La valve (1a, 1b) selon la revendication 8, **caractérisée en ce que** :
ledit premier axe (13a, 13b) et ledit deuxième axe (15a, 15b) coïncident sensiblement,
ledit quatrième axe (14a, 14b) est sensiblement perpendiculaire audit premier axe (13a, 13b) et audit deuxième axe (15a, 15b), et
ledit troisième axe (16a, 16b) est sensiblement perpendiculaire au plan défini par ledit premier axe (13a, 13b), ledit deuxième axe (15a, 15b) et ledit quatrième axe (14a, 14b).

10. Valve (1a, 1b) selon l'une ou plusieurs des revendications 1 à 9, **caractérisée en ce que** l'un ou l'autre, ou les deux, dudit élément de régulation de débit (7a, 7b) et dudit corps de valve (2a, 2b) sont réalisés dans un matériau plus souple et plus élastique que le matériau avec lequel est réalisé l'autre élément parmi ledit élément de régulation de débit (7a, 7b) ou ledit corps de valve (2a, 2b).

11. La valve (la) selon l'une ou plusieurs des revendications 1 à 10, **caractérisée en ce que** ladite protubérance profilée (9a) présente une géométrie sensiblement circulaire autour dudit troisième axe (16a) avec au moins deux protubérances latérales semi-cylindriques (50a) alignées parallèlement audit troisième axe (16a) et **en ce que** la géométrie dudit évidement profilé (10a) est complémentaire de ladite protubérance profilée (9a), avec au moins deux sièges latéraux (51a) qui correspondent auxdites au moins deux protubérances latérales (50a) ;
l'accouplement desdites au moins deux protubérances latérales (50a) avec lesdits au moins deux sièges latéraux (51a) définit lesdits au moins deux crans mécaniques.

12. La valve (1a) selon la revendication 1, **caractérisée en ce que** lesdites au moins deux protubérances latérales (50a) comprennent une pluralité de protubérances latérales (50a) disposées autour de ladite protubérance profilée (9a) selon un agencement circulaire à pas constant de manière à définir une pluralité de crans mécaniques à pas constant ; ladite au moins une position de fonctionnement comprenant une pluralité de positions de fonctionnement dudit élément de régulation de débit (7a) qui correspondent à au moins une partie de ladite pluralité de crans mécaniques à pas constant.

13. La valve (1a) selon la revendication 12, **caractérisée en ce que** ladite pluralité de positions de fonctionnement comprend :
- une première position de fonctionnement, dans laquelle ladite au moins une première entrée (3a), ladite au moins une deuxième entrée (4a) et ladite au moins une sortie (5a) sont reliées hydrauliquement à ladite chambre (6a) ;
- une deuxième position de fonctionnement, dans laquelle ladite au moins une première entrée (3a) et ladite au moins une sortie (5a) sont reliées hydrauliquement à ladite chambre (6a), tandis que ladite au moins une deuxième entrée (4a) est isolée hydrauliquement ; et
- une troisième position de fonctionnement, dans laquelle ladite au moins une deuxième entrée (4a) et ladite au moins une sortie (5a) sont reliées hydrauliquement à ladite chambre (6a), tandis que ladite au moins une première entrée (3a) est isolée hydrauliquement ;
et **en ce que** ladite au moins une position fermée comprend :
- une première position de fermeture, dans laquelle ladite au moins une première entrée (3a) et ladite au moins une deuxième entrée (4a) sont reliées hydrauliquement à ladite chambre (6a), tandis que ladite au moins une sortie (5a) est isolée hydrauliquement ; et
- une pluralité de deuxièmes positions fermées, correspondant à des positions intermédiaires entre lesdites positions de fonctionnement et ladite première position fermée, dans lesquelles ladite au moins une première entrée (3a), ladite au moins une deuxième entrée (4a) et ladite au moins une sortie (5a) sont isolées hydrauliquement.

14. La valve (1b) selon l'une ou plusieurs des revendications 1 à 11, **caractérisée en ce que** ledit évidement profilé (10b) présente une forme géométrique qui est sensiblement un arc d'anneau et **en ce que** ladite protubérance profilée (9b) est logée avec du jeu dans ledit évidement profilé (10b) de manière à permettre des rotations relatives entre ledit élément de régulation de débit (7b) et ladite chambre (6b) qui sont égales à la différence entre l'étendue dudit arc d'anneau et l'épaisseur de ladite protubérance profilée (9b) le long de la circonférence moyenne dudit arc d'anneau.
